# EUROPEAN PATENT APPLICATION

(11) **EP 2 676 682 A1**
(43) Date of publication of application: **25.12.2013**
(21) Application number: 11813585.4
(22) Date of filing: 30.11.2011
(51) Int. Cl.: A61L 15/28, A61L 15/58, A61L 24/00, A61L 24/08, A61L 31/04, A61L 31/14, A61L 31/16

(54) **THIXOTROPIC BIOLOGICAL ADHESIVE FOR USE IN INTERNAL BODY CAVITIES**

(30) Priority: 08.02.2011 US 201161440429 P
(71) Applicant: Cueto García, Jorge, C.P. 11700 México D.F. (MX)
(72) Inventor: Cueto García, Jorge, C.P. 11700 México D.F. (MX)
(74) Representative: Dennemeyer & Associates S.A.
(86) International application number: PCT/MX2011/000146
(87) International publication number: WO 2012/108753

(57) **Abstract**

A biological thixotropic adhesive that contains dextrin, and at least a structural component that provides thixotropy to the adhesive , and optionally at least an antibiotic that is very useful to stimulate the process of healing of tissues in a patient , for example, to prevent anastomotic leaks of he gastrointestinal system, to affix a prosthesis in inguinal hernioplasties, in a patient and to oclude a fistula in a patient. The biological thixotropic dahesive also has anti-inflammatory properties and can be used with a fatty tissue patch.

## Description

### FIELD OF THE INVENTION

This invention is related to a biological thixotropic adhesive for surgical use in humans, and particularly to a biodegradable adhesive, non-toxic, that seals the anastomosis of the digestive system providing temporary protection and preventing dehiscences and their lethal and serious complications. The present invention is related with a thixotropic adhesive to fix prosthesis in inguinal hernioplasties and for fistula closure. Additionally, this invention is related with a thixotropic adhesive with anti-inflammatory effect that can be used advantageously with a fat pad.

### BACKGROUND OF THE INVENTION

The capacity to unite biological tissues and/or to protect these unions (i.e., "anastomosis") has been an area of very important research for the biomedical investigators for several decades.

One of the greater problems that plague patients and surgeons world-wide is proper healing of an anastomosis within the gastrointestinal system and those of other systems as well. Dehiscence or faulty healing of the anastomosis may occur even though the anastomosis is well indicated, in the appropriate patient, and is adequately performed. Some dehiscences carry a high mortality rate (30-50%) and their complications require urgent reoperations and prolonged hospitalization in the intensive therapy units involving, for example, parenteral nutrition, complicated respiratory care, prolonged and costly antibiotic-therapy, numerous laboratory tests, etc.

At the present time, obesity has reached epidemic proportions throughout in the world, in developed as well as in underdeveloped countries. For example, in the United States of America one of every three adults is obese and approximately 5% suffer from morbid obesity, i.e., a body mass index (BMI) of 35 kg/m² or greater, and, what is worse, 1 of each 6 children suffers from obesity ^{1,2}. Although numerous medical treatments exist, these treatments only aid those patients with lower degrees of obesity, or in the short term, patients with morbid obesity, since, although initially the patients loose weight, a follow-up 10 to15 years after the treatment demonstrates that 98% of the patients have returned to their initial levels of obesity ("bounce"). Thus, they remain obese and frustrated. ¹⁻⁴ Morbid obesity is recognized at the present time as a chronic, incurable multi-factorial disease, the effects of which damage several organ systems that not only decrease the duration of life, but also the quality of life as well. besity affects the organism through the so-called co-morbidities, the most important ones being diabetes mellitus II, arterial hypertension, ischemic accidents of the heart and the cerebral circulation, serious pulmonary complications such as sleep-apnea, pulmonary disease of the obese, thromboembolic accidents, gastroesophageal reflux, several osteo-articular disorders, alterations of fertility, urinary incontinence, dislipidemias, etc¹⁻⁶. Moreover, objective evidence exists that obesity is a predisponent factor for the development of several malignant tumors such as breast cancer, colon-rectal cancer, prostate cancer, endometrial cancer, myelomas, leukemias, etc. ¹⁻⁴

Since the world-wide consensus meeting of 1991 in the United States of America, at the National Institutes of Health (NIH), ⁴ has been concluded that surgery is the only effective method for the control of the complications of obesity. With the introduction of the laparoscopic surgery everywhere in the world, the number of procedures of Bariatric surgery has increased in exponential form. But, due to economic reasons in some regions of the world, it is still practiced in conventional or open technique, which is also appropriate. ¹⁻⁶

The two main causes of mortality, major complications and enormous costs of gastric bypass, bilio-pancreatic diversions, etc., are the improper healing of the anastomosis called dehiscence (a premature bursting open or splitting along a surgical suture line, like in the junction or connection between the ends of the intestine, or the stomach pouch and the intestine as in the gastric bypass) and pulmonary thromboembolism ⁵⁻⁸. The prevalence of the first varies from 1 to 5% in general, it being less in some groups with more experience ⁵⁻¹³. The mortality caused by the anastomotic dehiscence ranges from 30 to 50%. Serious peritonitis ensues in patients that are already complicated with pulmonary, circulatory problems, etc., and require immediate and urgent reoperations, as well as special treatment in an intensive care unit, as mentioned before, which is extremely expensive. Thecost for the patients who survive is approximately $ 100,000 USD ¹². If 200,000 annual procedures are practiced in the USA, and if the prevalence of leaks is 2% (that at general level is greater), this would mean that the cost of these complications would be more than 20 million dollars annually, without taking into account indirect costs of the family, medico-legal and others.

This feared anastomotic dehiscence also occurs in surgery of the colon and rectum. The complications of diverticular disease are more and more frequent in patients older than 50 years (considering that life expectancy continues to increase everywhere in the world), like ischemic accidents, malignant tumors, volvulus, etc. The prevalence of dehiscences in anastomosis of the colon and rectum is greater than in Bariatric surgery and ranges from 5 to 15% of the cases reported in general. Also, mortality in these patients is greater, and the expenses in the intensive care unit are also very high for obvious reasons ^{14 -21}. Colonic dehiscences, in addition, diminish survival rates for patients operated on for cancer ²². When these patients are operated in elective or emergency situations, it is necessary to extirpate a segment of intestine. In debilitated, older patients, frequently with peritonitis, it is not possible to practice a primary anastomosis, but instead it is necessary to perform a colostomy or ileostomy. olostomies save many lives but nobody likes them. They require special care in all patients, and almost all patients want to be re-operated on so that the intestine can be re-connected and they can live normally. But, that also means another surgery, additional risks, expenses, etc.

Because endoscopic surgery is already accepted internationally as adequate for colon cancer treatment ^{16.21}, as for well as non-malignant diseases ¹⁷⁻²⁰, the number of these procedures performed will increase, as happened with cholecystectomy, antireflux surgery, nephrectomy for renal donor and many other procedures.

In the esophagus, the situation is more serious than in the colon, since the organ does not have a serous layer and anastomotic leaks (for example, in resections for tumors, congenital diseases, caustic burns, antireflux reoperations, etc.) produce mediastinitis with very high mortality rates. ²³⁻²⁵

It was mentioned previously that anastomosis can present dehiscences in spite of being well indicated, well done manually, with staples or using a mixed technique, without tension, with good blood supply, with good nutritional status, without peritonitis, regardless of being elective or urgent. It is very important to mention that dehiscences appear between the second and tenth postoperative day, but 98% occur between the second and sixth post operative day. ^{26,27}

During the 2^{nd} thru the 8^{th} postoperative day, the anastomosis does not have any strength by itself. In this time period, collagen deposition and new tissue bridges have not yet been built across the two ends (2^{nd} phase of healing process). As a result, the anastomosis is very weak, inflamed due to the presence of sutures and staples (foreign bodies) and bacteria, clots, etc. It is precisely during this time period that anastomosis leaks (or failures) often occur with the ensuing very grave and often lethal complications.

Anastomotic dehiscences, like a perforated duodenal ulcer, initially produce a discharge of gastro-intestinal secretions that cause an intense, localized inflammatory reaction called "chemical peritonitis." Later on, the discharge process continues and more secretions and bacteria are discharged producing secondary peritonitis, a most feared cause of mortality. ²⁷⁻²⁹

In the case of peritonitis by perforation and/or dehiscence of the colon, the peritonitis is of fecal type from the beginning and therefore of greater gravity in patients that, e.g., due to their age, other cardio-pulmonary ailments, metabolic complications, malnourishment, etc., have a greater surgical risk from the outset. ¹⁷⁻²⁰

In the esophagus, something similar happens with the discharge of diverse pathogenic germs in the mediastinum. Some groups such as Schardey et al. of Germany, affirm, based on their experimental and clinical observations, that "*....it can be prevented that the potentially pathogenic germs associated with the micro-leaks are in contact ... and produce these dehiscences.."* by means of the use of intensive antibiotic therapy. *^{23,24}*

A person knowledgeable in the area knows that in mammals in the healing process there are 3 main phases. The first phase starts immediately following the traumatic event with clotting accompanied by acute inflammation and, only after 7 - 8 days, the collagen deposition or second phase begins. The resultant scar is remodeled in the 3rd phase and then, there is considerable strength in this area weeks later. In the intestinal anastomotic area there is acute inflammation, clots, bacteria and secretions and it is known that during these days there is no collagen deposition in the edges. For this reason, the segments of intestine are held only by the sutures and/or staples, without bridges of new tissue to join both ends, but the anastomosis itself does not have any intrinsic firmness or strength per se. It is precisely in this critical period that most anastomotic failures or dehiscences occur. (Wasserberg N, Tzakis AG, Santiago SF, Ruiz Ph, Salgar ShK., Anastomotic healing in small bowel transplantation model in the rat, World J Surg 2004;28:69-73. ) . The method and adhesive herein described provide protection of the anastomosis during these critical days to prevent a dehiscence or failure to heal adequately.

In the case of fistulas that can be a consequence of an anastomotic dehiscence or trans-operative trauma, etc., they represent a very serious and annoying complication of various surgical procedures and some diseases of diverse organ systems like the gastrointestinal, respiratory, urinary, etc. When fistulas are not accompanied by obstruction or complicated with active suppuration, one can try to obliterate them with different methods or sealants with variable results ^{30 -33}.

In inguinal, incisional and other parietals hernias, the "gold standard" surgical treatment nowadays is called "without-tension" wherein a prosthesis or mesh is placed in the defect.³⁴⁻³⁷. Fixation of this prosthesis can be performed in several ways to avoid its displacement, both in open or laparoscopic surgery, frequently using sutures or staples. Staples are very expensive and, like sutures, they can incorporate, catch or compress small nerves, or produce osteo-condritis and post operative pain. Post operative neuritis is one of the frequent and important complications in inguinal hernia operations and, unfortunately, a frequent cause of reoperations and legal actions. The effect of fixation in this case is also temporary since after 4 to 5 days the prosthesis is included by the local connective tissue and no longer will be displaced ³⁷. A brief inspection in the surgical congresses and current literature shows that great interest exists to find an effective and safe method of affixing prosthesis in these situations. In fact, novel methods and alternatives are continuously described. ^{38,39}

US patent No 6,046,178 describes a composition for "treatment of external wounds" that includes a medication of starch hydroxylate like a maltodextrin to treat open wounds or skin defects as is the case of burns, ulcers and other types of cutaneous lesions that are exposed to contamination and conditions of the external environment. In a modality the starch hydroxylate it is mixed with the wounds fluids proteins to form a film that finally adheres to the subjacent tissues, being this impermeable to fluids and liquids.

In an effort to improve the wound healing process, the composition of patent 6.046, 078, it is applied as a gel, made of the mixture of maltodextrin with water and adding other gelatinizing agents such as , per example, glycerol. In patent 6,046,078, it is also described that water can be added to form a continuous phase of the emulsion, while the gelatinizing agent like glycerol, is mixed in the continuous phase in such a way that this dispersed phase forms a gel that has a final viscosity in the range of 29,000 to 37,000 cp. The gel composition presents several advantages and benefits such as , per example, an efficient delivery of dermatologic agents that have healing properties, and a deeper application of these agents in all areas of the wound. These benefits are provided by the viscosity of the composition, which necessarily requires the addition of glycerol as gelatinizing agent to provide the viscosity in the range of 29,000 to 37,000 cp. Glycerol is only recommended for external or topic applications due to its toxicity in the body cavities. In patent 6,046, 078 it is emphasized that a greater viscosity of the gel beyond the mentioned range, would produce slippage and could not adhere to the wound surface.

In an additional modality, US patent 6,046,078 contemplates the use of a dressing to reduce the bacteria innoculus of an infected wound, and inhibits the infection of a clean wound. For these finality the composition can be mixed with antibacterial Agents for the prevention or treatment of secondary infections. The dressing can be used in external environments, and being semipermeable permits the passage of gases and liquids, it can be said, allows breathing , m4° it needs to be changed several times daily. Also a pharmaceutical acceptable metal iron can be added us an additional component to this product to promote the development and growth of healthy tissue and thus promote the normal healing process. Inclusion of an additional component can improve the action all the film promoter agent combined with a monosaccharide. Optional components generally do not constitute more than 5 % of the composition.

US patent 6,046,178 does not describe nor suggests a sealing composition nor a thixotropic agent, non-toxic, for use in sterile vital internal body cavities, to seal and protect temporarily anastomosis of the digestive system (or other apparatus or body systems), nor to affix prosthesis in inguinal hernioplasties nor closure of fistulas where the composition of the adhesive or Sealant has a viscosity above 100,000 cp in resting state and a viscosity at the time of application in the range of 15,000 to 27,000 cp . US patent 6,046,178 does not teach a sealing nor a adhesive thixotropic non-toxic composition for use in internal sterile body cavities where does exist a 100% relative humidity and is completely isolated of the external environment (which means is not exposed to air current). Besides this, US patent 6,046,178 does not describe nor suggest a sealing composition thixotropic, non-toxic with a viscosity above 100,000 cp in a resting state that allows for stability during storage and to have a shel-time for over 2 years without any special conditions.

US 5,985,312 describes using metal compounds such as zinc oxide to enhance the bioadhesiveness of polymers used in drug delivery devices such as microspheres, tablets, capsules, which contain a drug or a diagnostic agent. Formulations are described in the form of microspheres as a mean of administering oral drugs where it exists the need to control or increase the absorption of pharmaceutical agents originated in polymeric devices such as polymeric microspheres for administering drugs through mucosal membranes. Mucosal membranes included are the gastrointestinal system. However the lumen of the gastrointestinal system is considered as external environment and is contaminated by very dangerous bacteria. The metallic compound is provided as a fine particles dispersion of an water insoluble metal oxide which is incorporated in the polymer or at least in its surface that is going to be adhered to the tissue surface. The fine particles of the metal oxide, can be produced by micronizing a metal oxide with the use of a mortar and pistil in order to produce particles that vary in size , for example , from 10.0 to 300 nm. The particles of the metal oxide can be incorporated in the polymer dissolving or dispersing such particles in a solution or dispersion of the polymer before forming the microcapsule, and then can be incorporating in the polymer during the formation of the microcapsule. The incorporation of the particles of the metal oxide over the surface of the microspheres, increases advantageously the capacity of the microsphere to bond or unite to the mucosal membranes and other tissular surfaces and improves the capacity to administer the drug of the microspheres. US patent 5,985, 312 does not describes nor suggest a sealing thixotropic agent non-toxic to be used in sterile internal body cavities to seal and penetrate through suture lines for temporary protection such high-risk suture lines like gastrointestinal anastomosis (or other apparatus or systems) and prevent leaks , nor to occlude fistulas, nor to affix temporarily prosthesis in inguinal parietal hernioplasties, where the composition of the sealing, adhesive agent has a viscosity above 100,000 in resting state and a viscosity at the time of application in the range of 15,000 to 27,000 cp . US patent 5,985, 312 does not teach a sealing nor a adhesive thixotropic non-toxic composition for use in internal sterile body cavities where does exist a 100% relative humidity and is completely isolated of the external environment (which means is not exposed to air current). Besides this, US patent 5,985, 312 does not describe nor suggest a sealing nor a thixotropic non-toxic composition with a viscosity above 100,000 cp in resting state that allows for stability during storage without any special conditions.

US 4,600,574 disclose a tissue adhesive in which a tissue-compatible material such as polysaccharide is combined with a solution comprising fibrinogen and Factor XIII. Due to the presence of fibrin, this adhesive suffers from the disadvantages as discussed further below.

US 5,496,872 disclose a non-toxic, biodegradable adhesive composition for surgical use. The composition contains a compound having at least two relative functions which can be used in combination with a biodegradable, synthetic or natural polypeptides such as polysaccharides.

Multiple efforts have been made to develop synthetic polymers, such as, for example, the cyanoacrylates, as adhesives and sealants. The tissue adhesive disclosed in U.S. Patent No. 3,667,472 (Halpern) relates to the surgical use of monomeric adhesives of C₂ - C₄ alpha-cyanoacrylate. This tissue adhesive cures on contact with water or blood to form a solid layer which crystallizes over the tissue. Nevertheless, a disadvantage of this class of adhesives is that it is contraindicated for application in internal organs or vascular surgery, and therefore in anastomosis, due to its toxicity and oncogenic effects which have been well documented ³⁰⁻³¹.

The well-known toxicity associated with synthetic adhesives has led investigators to develop biologically derived adhesives for use as union materials. of biological adhesive or glue is obtained from fibrin. Commercially, tissue adhesives of fibrin are derived from human plasma and thus raise potential risks to human health. Fibrin (and its derivatives) has been used in formulating biomedical adhesives with variable results from the experimental point of view and prospective studies in humans cannot be done for logical reasons. Its use for the protection of anastomosis has had apparently favorable results in a few reports. It is the only adhesive of use that is more or less accepted, but it is neither popular nor routine ³¹, ^{32, 41-43}. Nevertheless, fibrin has several disadvantages: risk of viral transmission like any other cryoprecipitate exists; use of fibrin requires processes for extraction of blood; costs associated with fibrin are high; it requires a special applicator; risk of allergic reactions is always present; and a fatality has been reported. Another disadvantage with fibrin is that the adhesion force is relatively weak compared to other adhesives.

More recently, combinations of products have been devised to be used as adhesives and tissue sealants. One such combination that has been described is the use of a combination of three substances prepared separately, i.e., the cryoprecipitate of human fibrinogen, thrombin in the presence of the calcium ion, and concentrated factor XIII, used to obtain a glue biomedical applications. Nevertheless, this type of product and systems of adhesives available do not avoid the health problems described before. Attempts have been made to isolate an analogous component that contains fibrinogen (to see, for example, Feldman, M. C., ET al., Arch Otolaryngol-Head and Neck Surg (1988) 114:182-185; Feldman, M.C., ET al., Arch Ophthalmologic (1987) 105:963-967; Feldman, M.C., ET al., M J Otology (1988) 9:302-305; Silberstein L. E., ET to, Transfusion (1988) 28:319-321). However, the use of preparations of the analogous fibrinogen also has obvious limitations.

Considering this, there is an acute need in the current state-of-the-art, and particularly in the daily surgical practice, to have a sealing, thixotropic non-toxic agent that allows for safe and efficient use in sterile internal body cavities to sealñ and penetrate through suture lines for temporary protection of high-risk sutures and anastomosis to prevent leaks of such anastomosis of the gastrointestinal system and other body organs and systems. Particularly it exists a need for a thixotropic biological non-toxic agent that permits temporary protection between the second and sixth postoperative day , non-toxic, and that does not present serious undesirable reactions and that will reduce to the maximum, the use of time and surgical resources and that has adequate tolerance with human tissues. Additionally, there is a need for biodegradable thixotropic agents that provide compatibility, stability , and an increased shelf-time compared to the products currently available and that promote a safer therapeutic option for the patient and reduce hospital stay.

A further need in the state of the art is that the same biomedical thixotropic adhesive used to seal and temporarily strengthen the anastomosis of the digestive system and other organs and systems, may be useful also to fix prostheses used in parietal hernias.

### SUMMARY OF THE INVENTION

Thus, in accordance with the invention, there is provided a technique involving a tissue adhesive or glue that protects and/or promotes the normal healing of tissues in human internal vital body cavities. This technique solves at least some of the problems associated with leaking of anastomosis and affixing prosthesis in inguinal and other hernia operations. Additionally, there is provided a composition for use in the above mentioned technique.

According to a further aspect of the invention, there is provided a biological thixotropic adhesive that includes mainly a dextrin as a dispersing agent, and at least a metal oxide, a component that provides thixotropy and increases the adhesiveness of the composition. Optionally the thixotropic agent can include at least an antibiotic. Based on the total weight, the thixotropic adhesive contains 80-97 % of its weight as dextrin dispersion, and the dextrin dispersion itself contains from 45-75 % of solid contents, and having a viscosity above 100,000 cp in resting state and an application viscosity in the range of 15,000 - 27,000 cp.

According to a further aspect of the invention, there is provided a method to stimulate the process of healing of a tissue in a patient that comprises applying a thixotropic adhesive that contains mainly dextrin and at least one structural component that provides thixotropy and increases the adhesiveness and, optionally, at least an antibiotic. Preferably the thixotropic adhesive contains dextrin as an element of dispersion and at least one structural component that provides thixotropy and increases the adhesiveness. Optionally, the thixotropic adhesive comprises at least one antibiotic. Based on the total weight, the thixotropic adhesive contains 80-97 % of its weight as dextrin dispersion, and the dextrin dispersion itself contains from 45-75 % of solid contents, and having a viscosity above 100,000 cp in resting state and an application viscosity in the range of 15,000 - 27,000 cp.

According to a further aspect of the invention, there is provided a method to prevent dehiscence of the anastomosis in a patient that comprises applying a thixotropic adhesive that contains mainly dextrin and at least one structural component that provides thixotropy and increases the adhesiveness and, optionally, at least an antibiotic. Preferably, the thixotropic adhesive contains dextrin as an element of dispersion and at least one structural component that provides thixotropy and increases the adhesiveness. Optionally, the thixotropic adhesive comprises at least one antibiotic. Based on the total weight, the thixotropic adhesive contains 80-97 % of its weight as dextrin dispersion, and the dextrin dispersion itself contains from 45-75 % of solid contents, and having a viscosity above 100,000 cp in resting state and an application viscosity in the range of 15,000 - 27,000 cp. According to a preferred embodiment, the wound is located inside the digestive system and the adhesive formula seals and protects against dehiscence of anastomosis of the digestive system.

In other aspect of the invention, there is provided a method to fix a prosthesis during the inguinal hernioplasties of a patient that comprises applying a thixotropic adhesive formula that contains mainly dextrin and at least one structural component that provides thixotropy and increases the adhesiveness. Optionally, the thixotropic adhesive comprises at least one antibiotic. Based on the total weight, the thixotropic adhesive contains 80-97 % of its weight as dextrin dispersion, and the dextrin dispersion itself contains from 45-75 % of solid contents, and having a viscosity above 100,000 cp in resting state and an application viscosity in the range of 15,000 - 27,000 cp. According to a preferred embodiment, the hernia operation is for an inguinal hernia.

In other aspect of the invention, there is provided a method occluding a fistula in a patient, that comprises applying a thixotropic adhesive formula that contains mainly dextrin and at least one structural component that provides thixotropy and increases the adhesiveness. Optionally, the thixotropic adhesive comprises at least one antibiotic. Based on the total weight, the thixotropic adhesive contains 80-97 % of its weight as dextrin dispersion, and the dextrin dispersion itself contains from 45-75 % of solid contents, and having a viscosity above 100,000 cp in resting state and an application viscosity in the range of 15,000 - 27,000 cp.

Considering these needs, an aspect of the present invention is to provide an adhesive thixotropic formulation or thixotropic adhesive based on a dextrin, which is safe and effective, and which has the following characteristics and properties:
I. The ingredients are non-toxic for application and use in sterile internal body cavities in human beings.
II. It reduces to the maximum leakage of gastrointestinal fluids. Due to the fact that maltodextrin has unique properties that seal porous structures, its use as an adhesive provides a better result over available agents and products.
III. The biological adhesive is thixotropic. This provides the capacity to remain stable as a solid product during long periods of time without any special storage conditions, and he time for its use it can be readily and quickly transformed into a gel for clinical use.
IV. Adherent or sticky. Due to its adhesive properties, the adhesive of the present invention, join tissues, including fatty tissue patches, though the formation of mechanical, chemical and/ or electrostatic bonds or links among them and tissue surfaces and seals the microscopic defects between sutures or staples. 44,47
V. Resistant to bacterial colonization. Dextrins have antibacterial properties that are useful for the protection of anastomosis where "micro leaks" could occur between the microscopic spaces that exist in sutures or staples, mainly discharges of secretions and bacteria.
VI. Biodegradable and safe. Although the adhesion produced by the maltodextrins can last for several weeks in the external environment, the human body has the capacity to metabolize them into simple monosaccharaides that are absorbed or eliminated easily without any toxic effects. In the US, the FDA and the National Research Council, have emphasized the use and inclusion of those biological adhesives in which there is a minimum use or organic acids and solvents, particularly when they are biodegradable.
VII. Has a pH very similar to the physiological within the range of 6-8.
VIII. Its viscosity in resting state is above 100,000 cp, whilst once conditioned for its application its viscosity is in the range of 15,000 - 27,000 cp.
IX. Anti-inflammatory effect;
X. Does not produce adhesions with neighboring intestinal loops
XI. Has an adhesiveness of 6Mpa (24 h)
XII. It has a TGA of 65.12 %

Therefore, another aspect of the present invention is to provide thixotropic compositions based on a dextrin as an effective, safe, biological adhesive, with appropriate adherent strength for biomedical applications, particularly those that involve smooth tissues. More specifically, the present invention is directed to useful thixotropic compositions in sealing and protecting temporarily anastomosis to eliminate dehiscences to the maximum, and that, in addition, can serve as an adhesive for inguinal prosthesis in hernia operations and for occluding certain types of fistulas.

In the present invention, another aspect of the thixotropic composition based on a maltodextrin, is to combine the maltodextrin with other agents or components that provide special and desirable properties and that avoid adhesions with neighboring organs and structures, for example, a patch of fatty tissue or collagen can be used for that purpose.

### BRIEF DESCRIPTION OF THE DRAWINGS

Various features and attendant advantages of the present invention will be more fully appreciated as the same becomes better understood when considered in conjunction with the accompanying drawings, in which like reference characters designate the same or similar parts throughout the several views, and wherein:
Figure 1 shows the results of a test to determine the hydraulic pressure required to produce a leak ("bursting pressure") in suture lines in the rat's intestine, Phase 1. (Test ANOVA);
Figure 2 is a microphotograph of a segment of the intestine of a rat from group 1 that died in 3 days, in which the defect was covered with a patch, but not an adhesive or suture, Phase 2;
Figure 3 is a microphotograph of a segment of the intestine of a rat from group 1 that survived 3 weeks, in which the defect was covered with a patch but not adhesive or suture, Phase 2;
Figure 4 is a microphotograph of a segment of the intestine of rat from group 2, in which a patch was placed with the biological adhesive of the present invention, Phase 2;
Figure 5 is a photograph showing the macroscopic aspect of the duodenum three weeks after placing a patch with adhesive in a dog, Phase 3; and
Figure 6 is a microphotograph showing the microscopic evaluation of the duodenal injury of a dog after three weeks, Phase 3.
Figure 7 is a microphotograph of an intestinal segment that shows the penetration of the adhesive 5 days after the application of a fat patch with the biological thixotropic adhesive of the present invention.
Figure 8 is a microphotograph of an intestinal segment that shows the process of reabsorption 17 days after the application of a patch with fatty tissue with the biological thixotropic adhesive of the present invention.
Figure 9 is a microphotograph of an intestinal segment that shows the complete penetration in the intestinal wall, 11 days after the application or a fat patch with the biological thixotropic agent of the present invention.
Figure 10 is microphotograph of an intestinal segment tha shows the inflammatory reaction of a suture ("foreign body") in a suture without the application on the biological thixotropic adhesive of the present invention.
Figure 11 is a microphotograph of an intestinal segment that shows giant cells as a chronic inflammatory response in the serosa with a fat patch without the application of the biological thixotropic adhesive of the present invention, 17 days after the operation.
Figure 12 is a microphotograph of an intestinal segment that shows the penetration of the biological thixotropic adhesive of the present invention , 11 days after the operation, without an inflammatory reaction even with the presence of a foreign body.
Figure 13 is a microphotograph of an intestinal segment that shows the penetration of the biological thixotropic adhesive of the present invention 8 days after the operation. There is no apparent inflammatory response.

### DETAILED DESCRIPTION OF THE INVENTION

The term "dehiscence" used presently includes any defect or failure of the anastomosis in the gastrointestinal, respiratory, urinary systems, etc., that can produce leakage of secretions and bacteria through this defect, with very serious and frequently lethal consequences. This statement is not to be considered limitative but rather illustrative of some of the applications of the biological adhesive of the present invention to eliminate dehiscences of anastomosis to the maximum.

The terms "adhesive formulation" and "adhesive" are used in an interchangeable indistinct way and mean any biological thixotropic adhesive that offers protection.

The terms thixotropy and thixotropic as used in the present invention, are related to the physico-chemical property of some non-newtonian fluids pseudoplastics that show changes in itheir viscosity related to time , that is to say, that the more that mechanical or hermic energy is applied, the more the viscosity decreases.

The adhesive thixotropic formulation of the present invention is biodegradable, non-toxic, safe and effective, and has the capacity to provide temporary protection of anastomosis for several days, particularly during "the critical days" in order to enhance healing. It must be understood clearly that the adhesive formulation of the present invention is not a substitute for good surgical techniques, but an additional element of protection.

In an aspect of the present invention, the adhesive formulation is constituted mainly by a natural product, and which is easy to use and inexpensive. The formulation has demonstrated to have very useful properties in experiments made in minor and major animal species, and is very well tolerated and biologically degraded, (depending on the physico-chemical mixture and concentration of additional components), after the 10th post operative day without any collateral or undesirable effect. In particular, the adhesive formulation has demonstrated that it penetrates through the suture lines and even quicker thought the suture holes, and in a few days it moves along the total thickness of the intestinal wall and reaches the mucosal level where it begins the reabsorption process.

In another aspect of the invention, the adhesive formulation can be used with such similar results for temporary fixation of prosthesis in inguinal hernia operations (and others as well). In addition, the thixotropic adhesive can be used to facilitate the closing of some digestive fistulas or fistulas of other organ systems, if there is no obstruction or active suppuration.

In agreement with the previous statement, the biological thixotropic adhesive of the present invention has the characteristics and properties, such as, it allows effective temporary protection of the anastomosis between 2° and 10° postoperative days, is not toxic, and does not produce adverse reactions or risks of transmission of infectious agents. It minimizes the demands on surgical resources and time, and demonstrates superior biocompatibility. In addition, the biological thixotropic adhesive of the present invention offers improved convenience and permanence compared to formulations presently available. Also, the inventive formulation, promotes treatment of the patient, and reduces hospital stays and/or medical supervision. Additionally, the strength of adhesiveness of the present invention does not affect the normal physiological functions of the digestive system and other organ systems, where it is applied.

An important advantage of the inventive biological adhesive is that the adhesive effectively can protect the anastomosis during the crucial time period between the 2nd and 8^{th} postoperative days. It is during this time period that the anastomosis is particularly week because collagen deposition and development of new tissue bridges (2^{nd} phase of healing process) has yet to occur.

Another important advantage of the inventive biological thixotropic adhesive is its non-toxic nature, and presents an anti-inflammatory effect thereby allowing the formulation to be used in internal body cavities as an internal biological adhesive for anastomosis, without the safety concerns associated with, for example, biological adhesives obtained from fibrin that are very expensive, and complicated to use. Additionally, in this time of cost containment, the biological thixotropic adhesive of the present invention is extremely inexpensive.

Once again, this biological thixotropic adhesive contains a material of fundamentally natural origin that has the property to adhere to tissues and/or live structures (call "bio-adhesion") for a determined period of time. So, in order for this "bio-adhesion" to be effective, an intimate contact between the adhesive material and the receiving tissue must exist. Preferably, the biological thixotropic adhesive makes not only direct contact with the surface of the tissue and/or live structure, but also penetrates into the hollows or grooves of the receiving tissue so that mechanical, chemical and/or electrostatic connections or unions or links are formed. Of course, the adherent property of the biological thixotropic adhesive will be affected by certain factors, such as the physical and chemical conditions where it is applied ⁴⁴⁻⁴⁷.

The biological adhesive of the present invention includes a dextrin, structural component that provides thixotropy and increases adhesiveness and optionally an antibiotic. Each one of the components of the biological adhesive is present in suitable amounts to provide the characteristics and properties previously mentioned.

The term "dextrin" as it is used in the present invention means a glucose polymer that forms from starch hydrolysis and which has glucose units connected by α-1,4 or α-1,6 links. As it is known, dextrins are hydrosoluble polysaccharides (dextrorotatory polymers) of diverse molecular weight and chemical structure, obtained from partial hydrolysis of starch. In biological systems, this conversion takes place by the enzymatic action of α-glycosidases or dextrinases, but industrially the conversion is carried out by means of acids, heating or both. The dextrins are not susceptible to fermentation, and have antibacterial properties. Dextrins exist with a high or low level of conversion (hydrolysis). In general, the former are more hydrosoluble, whereas with greater solid concentration (for example, borax, as discussed below) the greater the adhesiveness. Dextrins also exist naturally in some vegetables during the process of germination and maturation. The dextrins also can be classified as white dextrins (greater viscosity), yellows (greater adhesiveness) and "British gum", which have a high degree of conversion. Preferably, the dextrin has a dextrose equivalent (ED) of 10-11 and a pH in a solution at 20 % of 4 - 7 . Additionally, the dextrin in a preferably presentation of is a white soluble powder slightly sweet, non-higroscopic with a density of 0.5-0.6 g/ml.

Preferred dextrins for use in the formulation of the biological adhesive of the present invention are those that exhibit a viscosity of 12,000-33,000 cp (for example, 15,000 - 32,000 cp, or 12,000 - 25,000 cp, or 15,000 - 25,000 cp, or 15,000 - 18,000 cp, or 30,000 - 32,000). In preferred embodiments of the invention, a dextrin such as maltodextrin is used at one of two different concentrations: (1) maltodextrin with a viscosity 12,000-18,000 cp (such as 15,000-18,000 cp), especially 12,000-16,000 cp, wherein the resultant composition is in the form of a suspension which is easy to apply and has the above mentioned properties, and (2) another maltodextrin with a higher viscosity (e.g., 30,000 -32,000 cp), wherein the resultant composition is in the form of a paste. These viscosity ranges are based on the formulation containing dextrin, the carrier (e.g., water), and an adhesiveness modifier (e.g., borax or zinc oxide). It is believed that the antibiotic will have little effect on the viscosity.

In embodiment (1) mentioned above, the resultant adhesive composition needs between about 10 - 12 minutes to cure (based on animal tests) and produce the desired adhesiveness. In embodiment (2) mentioned above, the resultant adhesive composition has a higher concentration of zinc oxide and/or borax and thus requires only 5 - 6 minutes to cure (based on animal tests), but due to this characteristic the composition is applied as a paste and produces minimal adhesions to surrounding fatty tissues. As noted, the estimated curing times mentioned above are based on acute animal experiments wherein curing times were measured in the determination of the bursting pressure of a suture line. In clinical use in humans, the adhesive formulation will obviously be allowed to sufficiently cure and will remain in place for several dates protecting the anastomosis, or affixing the prosthesis, or occluding the fistula, and then later on will be metabolized.

In the formulation, the dextrin is used in the form of a mixture or suspension with a pharmaceutically acceptable carrier/excipient such as water. This mixture/suspension can have a solids content of, for example, 45-75 %, preferably 55-70, especially 60-68%.

The addition of some solids to the dextrin, for example, borax hydroxide, iron, zinc, etc., modifies certain properties of the dextrins such as viscosity, drying time and adhesiveness.

The dextrins have been used commercially in diverse forms for several decades, like as adhesives for paper, pasteboard, packages, etc. In spite of daily frequent contact with the skin and human digestive system, no toxic effects are known. In fact, certain types of dextrins, for example, malto-dextrins, are used for the manufacture of beer.

The selection of the dextrin as the main component of the present inventive adhesive formulation or biological glue - for use as safe, temporary protection of anastomosis of the gastrointestinal, respiratory, urinary systems, and to fix the prosthesis of hernia operations among other clinical uses - is based on several months of extensive tests with diverse products, on a large number of inorganic materials and, later on, derived from experimental work in minor and major animal species, under a strict research protocol. As mentioned previously, the selection of dextrins is based, in addition, on the following properties and/or characteristics:

### Non Toxic for Humans

After several decades of commercial use of dextrins in adhesives, with daily, direct contact to the skin, the mucosa and the lips, etc., no toxic or adverse effects are known. In addition, an extensive literature search does not reveal any evidence of toxicity. High-residue foods such as complex carbohydrates, including dextrins and similar compounds, are ingested daily by human beings without any toxicity, and related compounds (maltodextrins) are used in the production of the beer. They are also used in the manufacture of capsules, etc.

Recently, sulphated dextrins have been used successfully in humans by a group of investigators at Hammersmith Hospital of London, wherein sulphated dextrins were administered intraperitoneal to patients with Kaposi's Sarcoma, since they have the property to inhibit the angiogenesis in this tumor. A favorable response by its use was observed without undesirable effects, i.e., they were well tolerated, even in these very sick patients ⁴⁸.

Use of icodextrins is common in peritoneal dialysis in concentrations from 4 to 7% ^{49,50}. An example of the use of icodextrins is disclosed in U.S. Patent No. 6,770,148, issued 3 of August of 2004, and entitled "Solution for peritoneal dialysis that contains modified icodextrins". Other groups recently have used intraperitoneal icodextrins in women with reproductive problems. Apparently, pelvic adhesions decreased and no collateral or toxic effects were observed, only a single report of a minor allergic reaction ^{51,52}.

Cyclodextrins have special properties that allow them to improve the stability, solubility and bioavailability for oral absorption of some drugs, and they are used accordingly. Cyclodextrins are degraded enzymatically in the digestive lumen, mainly in the colon, and no toxic effects are known. Due to the mentioned properties, use of cyclodextrins intravenously is under investigation at the present time with great interest 53

Maltodextrins also are used orally in solutions for rehydration, and in pill manufacture, etc., without any reports of toxicity. ⁵⁴

Finally, it must be remembered that a compound closely related to dextrin from a biochemical point of view, that is, a modified starch (hydroxy-starch), is frequently used intravenously in emergency situations in humans as a volume-expander. ⁵⁵

In the studies discussed below, maltodextrin is used. Maltodextrin, derived from vegetables and, in industrial processes, mixed with borax (2-4 %), has been used as glue commercially for decades. However, since other dextrins share similar physic-chemical properties, the applicant considers that they can also be used for similar purposes in accordance with the invention. In fact, they are used frequently in humans in different medical applications successfully. Maltodextrins are non-toxic and are used commercially in the food-processing business. They are ingested daily in many places in the world and are easily digested in the GI tract to form glucose.

### Resistant to Bacterial Invasion

Dextrins have antibacterial properties. This property is of particular importance in biological adhesives for protection of anastomosis where the "micro leaks" of secretions and bacteria between microscopic spaces of sutures or staples can occur, where germs make contact with organs and intraperitoneal and/or intrathoracic structures, or with foreign bodies like a prosthesis with very serious consequences.

### High Penetration in Hollows or Grooves

The application of dextrins in porous substrates such as paper and cardboard, has demonstrated their high capacity to penetrate hollows or grooves on and through surfaces to which they are applied. Therefore, penetration into grooves or hollows of the receiving tissue is an important property of dextrin, with respect to microscopic defects in the anastomosis (denominated "micro leaks" of liquids and bacteria present in the lumen of the digestive system).

### High Resistance to the Humidity

Adhesives based on dextrin exhibit a high environmental resistance to humidity. This characteristic is important because the adhesive can thus be applied in an internal cavity with normally high humidity. Also, the dextrin will work as an adhesive at the human body temperature.

### Biodegradable

Although in an external environment the strength or force of the adhesion can persist for many weeks or months, dextrin's force or strength of adhesion in the human body is temporary, due to the capacity of living human tissues to metabolize the adhesive. The resulting products are simple carbohydrates that are eliminated or absorbed without adverse consequence. When metal oxides are used to increase the viscosity and the tack, the adhesive of the present invention can be observed to adhere to the intestinal segment for up to 3 weeks later. In this case, if an interphase is not used, some adhesions to the neighboring intestinal loops may occur.

### Very Inexpensive

The cost of the adhesive according to the invention is approximately, $2,000.00 pesos ($ 200 USD) per 60 kg.

Preferred dextrins (in admixture with a pharmaceutically acceptable carrier/excipient such as water) for use in the formulation of the biological adhesive of the present invention are those that exhibit a viscosity of 12,000-33,000 cp such as 15,000 - 32,000 cp, or 15,000 - 25,000 cp, 15,000 - 18,000 cp, or even 30,000-32,000 cp (higher zinc oxide concentration). The latter ones with higher zinc oxide and/or borax content (this due to the fact that the industrial production includes borax to increase viscosity and tack), can also be used, but topical application requires an extra 2 minutes for it is a paste. The dextrin-water mixture (having a solids content of, for example, 45-75 %, preferably 55-70, especially 60-68%) is present in the formulation of the biological adhesive in an amount of about 80% - 97% (e.g., 90-97% or 80%-95%), preferably 92% - 96%, by weight of the total formulation.

In the less viscous form (viscosity of, e.g., 12,000-16,000 cp), the solid content of the dextrin/water mixture is, for example, 60-63 %, and it has a pH of 8-9 with a tack time of about 10-14 min. This formulation works well at room temperatures (e.g., 20-30 °C), does not require heating and remains active for 6 months in a dry storage room. The more concentrated viscous forms having a viscosity of 30,000 - 33,000 cp, is in the form of a paste, also works well in room temperature of 20-30 °C, and has a tack time of 4-6 min.

The structural component that provides the thixotropy and increases the adhesiveness used in the biological adhesive of the present invention is preferably an insoluble metallic oxide powder. As it is known in the art, borax is used in industrial processes to modify the physical characteristics of dextrins such as, for example, viscosity, adhesiveness, solubility in the water, tack, etc. Nevertheless, the use of borax can produce adverse effects such as adhesion when it is in free direct contact with the intraperitoneal structures. It is known that diverse metal compounds such as calcium, iron, titanium, zirconium, and zinc can be incorporated into polymers to increase the capacity of the polymers to adhere to tissues. These metallic compounds preferably are insoluble in water and have an ionizable charge in the surface where they are used. Incorporation of the metallic compound into the polymer can be achieved by mixing with the polymer or by covering the polymer ("coating") ^{46,47}. Also, it is known that some polymers that contain metals actively adhere to tissues such as mesenteric, surrounding adipose tissue, a phenomenon we observed with very viscous dextrins (30.000 cp or higher) when the biological adhesive is placed in the peritoneal cavity without a patch or interphase of a biodegradable material.

Zinc oxide powder is a preferred structural component that provides thixotropy and increases the adhesiveness for use in the biological adhesive dextrin-based of the present invention. Zinc oxide has the approval of the FDA as a pharmaceutically acceptable additive and can be ingested by humans without adverse collateral effect. In addition, zinc oxide, due to its emollient, absorbent properties, among others, is daily used in an endless number of applications such as treatment of wounds, decubitus ulcers, "diaper rash" in babies, etc. As mentioned previously, zinc oxide when added to the polymer (dextrin) increases the biological adhesiveness of the polymer, its tack, and diminishes the drying time, thus improving the properties of the formulation as a biological adhesive to temporarily protect the anastomosis, or to fix a prosthesis used in hernia operations. With a higher viscosity, e.g., 30,000 cp (or higher), the biological adhesive is in the form of a paste which must be applied with, for example, a cotton tip applicator. Finally, zinc, as it is well known, is a very important ion in human metabolism and in the healing process.

The structural component that provides thixotropy and increases the adhesiveness is present in the biological adhesive of the present invention in a suitable amount to modify the adhesive strength of the adhesive in such a way that the biological adhesive seals and remains temporarily in the anastomosis. In addition, the amount of adhesiveness exhibited by the biological adhesive is suitable to promote healing of the anastomosis. The structural component that provides thixotropy and increases the adhesiveness is present in the formulation of the biological adhesive in an amount up to about 19 % by weight, but preferably is about 8 - 12 % by weight of the total formulation, and very preferably the structural component that provides thixotropy and increases the adhesiveness is present in an amount of about 9 to 11 % by weight of the total formulation. Also, the cost of zinc oxide is very inexpensive, similar to the cost of dextrin.

In an embodiment particularly preferred, the thixotropic non-toxic agent of the present invention, is a thixotropic viscous gel with a viscosity between 17,000 to 25,000 cp, a neutral pH 6.5 - 7.5 , a TGA of 61.12 and an adhesiveness at 24 h of 6 Mpa. The TGA -in English Thermal Gravimetric Analysis-, is a commonly employed test in research to determine the characteristics of of materials such as polymers, in order to find out the degradation temperatures, etc.

Because there is solid experimental and clinical evidence that extensive bacterial invasion interferes with the healing process everywhere, the adhesive formulation of the present invention can include optionally an antibiotic, preferably to avoid problems associated with bacterial invasion. The inventor of the present invention has found that the preferred antibiotic agent for topical use is kanamycin due to its well-known efficacy when used topically in the surgical wounds but more importantly because it has been used safely and extensively inside the abdominal cavity.

It has been known for many years, that the topical use of intraperitoneal kanamycin in doses of 2-3 grams applied at the site of maximal bacterial contamination (for example, in the case of a perforated appendix, it is applied near the site of the stump) during surgery, may help prevent residual septic complications ^{56 -58}. Recently, its successful use in topical form in the large incisions of bariatric surgery - known for high risk for infection -has been reported, without adverse effects ^{59,60}. Kanamycin, used clinically by the intramuscular route, in doses of from 1 to 2 g/day during 7 to 10 days, has demonstrated in time to be an effective and safe antibiotic. However, for the required antibiotic effects in the adhesive formulation of the present invention, the antibiotic agent is used in amounts that do not interfere with the healing process. Preferably, the antibiotic agent is present in an amount of between 0.1 and 1% by weight of the total formulation (for example, of 100 to 125 milligrams). In a particularly and preferable modality of the invention, the antibiotic agent is present in amounts of between 0.2 and 0.5 % in weight of the total formulation (for example, of 250 to 500 milligrams single dose in the topical application).

Other antibiotics are also suitable for use in the inventive composition such as a 1^{st} generation cephalosporins like cefazolin. This popular and inexpensive antibiotic is used topically and subcutaneously to prevent incisional infections. Ann R Coll Surg Engl. 1978 May;60(3):243-5.). Gentamycin, Clindamycin and Vancomycin are also suitable antibiotics. These agents are being used topically with collagen with good results externally. (Phinney, R.B., Schwartz, S.D., Lee, D.A., Mondino, B.J.: Collagen shield delivery of gentamycin and vancomycin. Archives of Ophthalmology, 1988. 106 pp. 1599-1604).

The formulation may also pharmaceutically acceptable adjuvants and excipients. In addition, the formulation may contain further active agents. For example, the formulation may contain growth factors, i.e. substances that in very small amounts can be placed in wounds to stimulate development of new vessels, namely ngiogenesis which is vital for the development of new tissue such as collagen. Such growth factors are derived mainly from macrophages present in the wound along with different types of cytokines in the initial phase of the healing process and some of them are the platelet one (PDGF), a vascular GF (VEGF), Keratinocyte GF (wounds), etc. (te Velde EA, Voest EE, van Gorp JM, Verhemm A, Hagendoorn J, Gebbink MF, et al., Adverse effects of the antiangiogenic agent angiostatin on the healing of experimental colonia anastomoses. Ann Surg Oncol 2002;9:303-9; Bennet NT, Schultz GS, Growth factors and wound healing: Part II. Role in normal and chronic wound healing. Am J Surg 1993;166:74-81).

A further example of an additional active agent is arginine. Arginine is an amino acid that plays a role in cell division, healing of wounds, improving immunity to illness, etc. It is used by the body to make nitric oxide, a substance that dilates blood vessels which plays an important role in wound healing (Rizk M, Witte MB, Barbul A. Nitric oxide and wound healing. World J Surg 2004;28:301-6).

L.P. Fielding el al described in the British Medical Journal the results of a large multicentric study in colo-rectal cancer surgery in which the concept of mini-leaks-which occur at a much higher rate than previously thought in these anastomosis- is supported, and where the clinically relevant anastomotic leaks produced three times as much mortality, expense and hospital stay ^{62,63}. Other reports have demonstrated that although an anastomosis that tested well during the operation and did not show a visible air or fluid leak (so-called physical impermeability), that does not mean that is completely sealed to prevent passage of bacteria and secretions into the peritoneal cavity (biological impermeability) as shown by A. Zaporozhets⁶⁴. In fact, mini-leaks not clinically relevant are shown in patients when radio-opaque enemas are administered in the first 7 days postoperatively and many of these leaks do not produce peritonitis. However, this may explain why some patients have a more torpid postoperative course, others develop a pericolic abscess later, and in some others an extensive adhesive process surrounds the area of the anastomosis.

In accordance with a method aspect of the invention, once an anastomosis has been finished and duly tested for its integrity, the biological adhesive of the present invention is applied on the entire surface of the anastomosis to seal and form a barrier, a film impermeable to the passage of bacteria, other cells , secretions, towards the peritoneal cavity (biological impermeability), for example with a sterile cotton tip applicator. In another embodiment of the present invention, it can be then applied a patch (an interphase) of cellulose or collagen as well as fatty tissue patches with sterile technique to cover the area in a way that adhesions with surrounding structures are avoided. Although in chronic animal experiments this method provides survival in defects not sutured, in humans once the anastomosis is completed and tested, the biological thixotropic adhesive of the present invention is preferably applied with an interphase to reinforce the suture lines, to promote healing during the critical period of several days when the anastomosis is held only by sutures and/or staples, in an area of acute inflammation, with no collagen deposition, without any intrinsic strength per se, and in a manner which will prevent the occurrence of a dehiscence and/or mini-leaks. This is a very advantageous aspect of the inventive adhesive and method. A similar method can be used to fix the prosthesis in inguinal hernioplasties.

### Anti-inflammatory properties of the biological thixotropic adhesive:

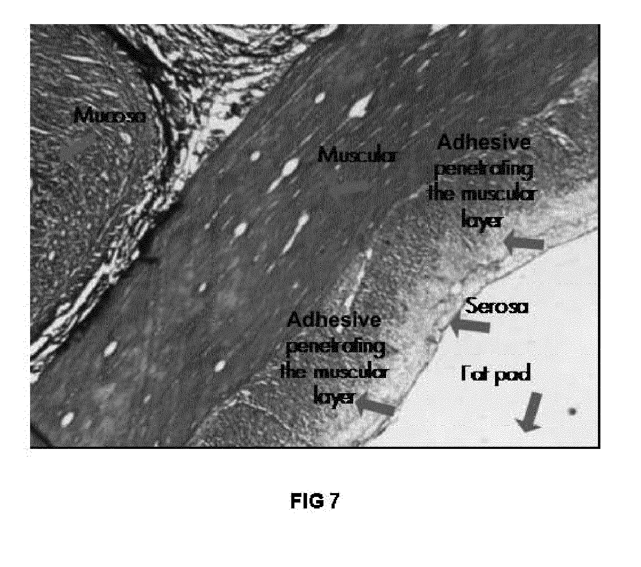

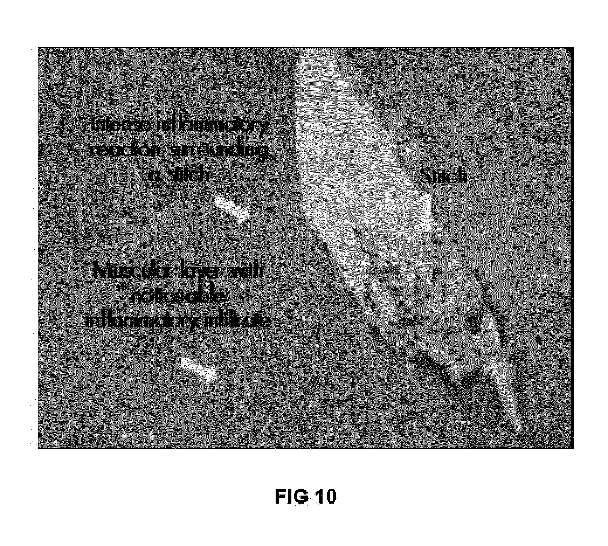

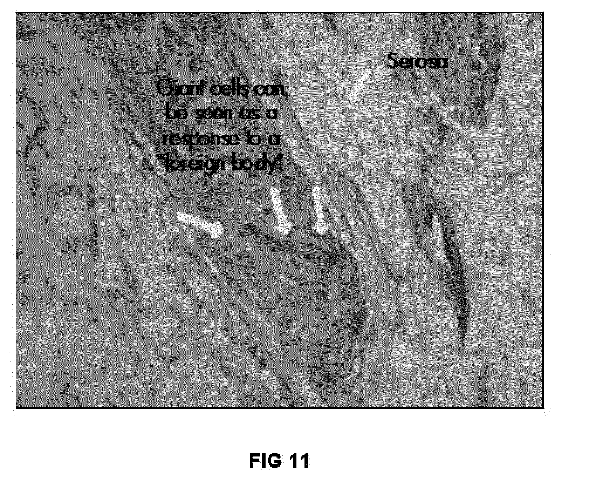

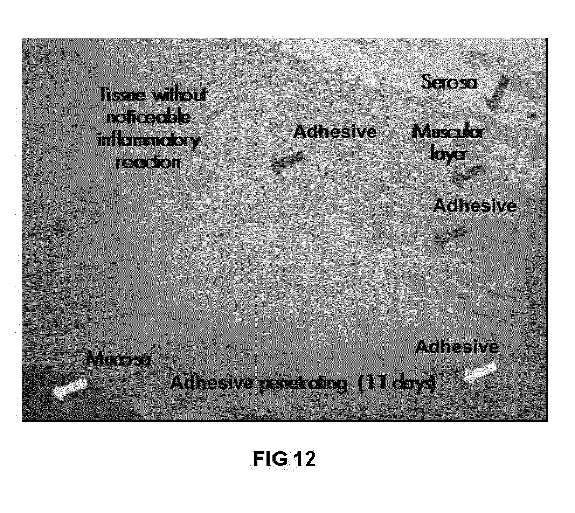

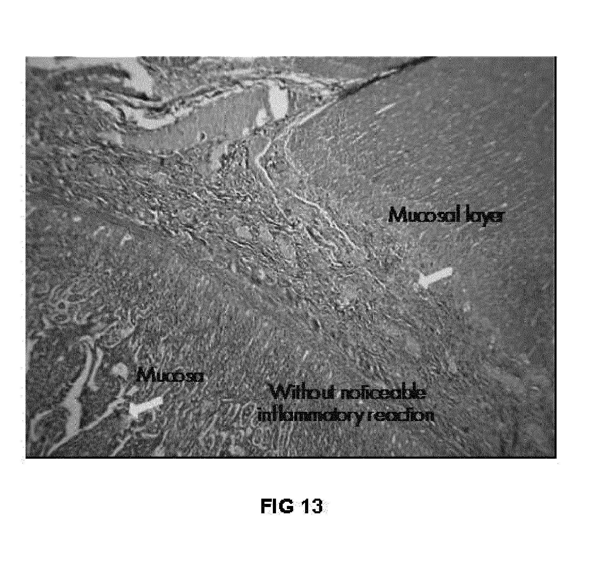

The biological thixotropic adhesive of the present invention, has a potent local anti-inflammatory effect in the intestinal wall as shown in Figures 7,12 and 13. 8 days after the application of the biological thixotropic adhesive, the product has penetrated to the mucosa and no inflammatory reaction is seen. (fig 12). In Fig 13 it can be seen that 11 days after the application of the biological thixotropic adhesive, that such adhesive is seen in the full-thickness of the intestinal wall without any inflammatory reaction, that is to say is perfectly well tolerated by these tissues. This is confirmed after comparing segments with and without the application of the biological thixotropic adhesive adhesive. In the histological sections without the adhesive (figs10,11), it can be seen that there is an intense inflammatory polymorphonuclear cell infiltrate (PMN s), a marked difference in a similar section of a suture line treated with the biological thixotropic adhesive that has penetrated the full-thickness of the intestinal wall. (see arrows) in which there is no inflammatory changes. Figure 10 shows an intense inflammatory reactio to a suture ("foreign body") without the application of the biological thixotropic adhesive of the present invention, 17 days after the operation.

In Figure 11 giant cells can be seen as a chronic inflammatory reaction near the serosa with fat patch in a dog without the application of the biological thixotropic adhesive of the present invention, 17 days after the operation. This can be a critical issue in clinical surgery in an anastomosis or in a suture , for it is well known that in septic peritonitis, the presence of infection interferes with the healing process and this can produce leak of intestinal contents into the sterile peritoneal cavity with its lethal, grave consequences for the patient.There is abundant Iterature confirming the deleterious effects of infection in the healing process.

### Clinical Use of the biological thixotropic adhesive at the time of surgery :

The biological thixotropic adhesive does not require refrigeration, or special storage condition, nor a specialized complex delaying time-consuming type of preparation prior to its use -it requires less than a minute with the use of any instruments or a liquid heating device 40 o C-, to transform it into a gel ready for application thanks to its special thixotropic properties , abd thus it can be applied into the anastomosis, a suture line, etc.

### Absorption tests of the biological thixotropic agent :

In dogs as in humans, basal and 1, 2 and 3 h blood samples have been obtained when the biological thixotropic agent to determine glucose and Zinc levels. It was observed a moderate transitory hiperglycemia that returns to normal in 1 h, and this is obviously related to the anestyhesia, initial surgical maneuvres, etc.,.Zinc levels remained unchanged. Similar changes were observed in sham operations (no adhesive applied).

All these findings reinforce the concept that the biological thixotropic adhesive acts ïn-situ¨, locally which will help for Sanitary Registration by the F.D.A. and other regulatory international entities for it will be registered as a Medical Device Type II short-term.

### Accelerated Shelf-time of the biological thixotropic agent:

These international approved tests are conducted in a specially designed container have shown that even without the need for refrigeration or any special storage conditions, , the adhesive remain useful for over 2 years.

### Bacteriological tests for the biological thixotropic adhesive :

Several surface and deep container samples were sent to the bacteriological laboratory for testing and all were reported as "sterile". In most instances these containers were left open in the "black area" of the laboratory where many animal procedures are done, and no special precautions were taken to avoid contamination, even after 7 days.

The biological thixotropic adhesive works with or without antibiotics.

Repeated acute and chronic tests in rats and dogs have recorded that the biological thixotropic adhesive works as protector of the suture lines with or without the addition of an antibiotic.

Both biological adhesive and interface covering the same, disappear in 3 to 6 days although there can be several days longer when using the higher viscosity adhesive. This is particularly useful in both anastomosis and inguinal plasties.

The authors recommend that after the application of the biological adhesive the anastomosis should be "sealed" with adipose neighboring tissue, as it is routine for many surgeons, since the described biological adhesive adheres strongly to said fatty tissue.

During different experimental phases in dogs and in rats, the biological thixotropic adhesive of the present invention, when applied with a fat patch in the suture lines, produced considerable increase in "burst pressure" starting in 15 - 20 minutes but mostly after several hours after the application. A remarkable finding was that after 7 days of the application, the fat patch could not be detached from the suture lines lest the intestinal wall was avulsed or lacerated occasionally even to the lumen of the bowel loop. In those suture lines without the adhesive, tha patch can be detached easily without any lacerations in the intestinal loop.

During these experiments it was also found that the solute concentration affected the initial tack time but not the properties dscribed hours after the application, namely that the more viscous the faster the tack time. The solute concentration also is important for it contributes to provide thixotropy which is critical for the homogenicity and stability of the biological adhesive. Zinc oxide also contributes importantly in the tack time and to the thixotropy. Additionally and very important, is the fact that the Zinc ion as is very well known, is fundamental in the process of healing in mammals as a structural component of the metallo-proteins. Zinc also has been used for decades in other curative areas clinically.

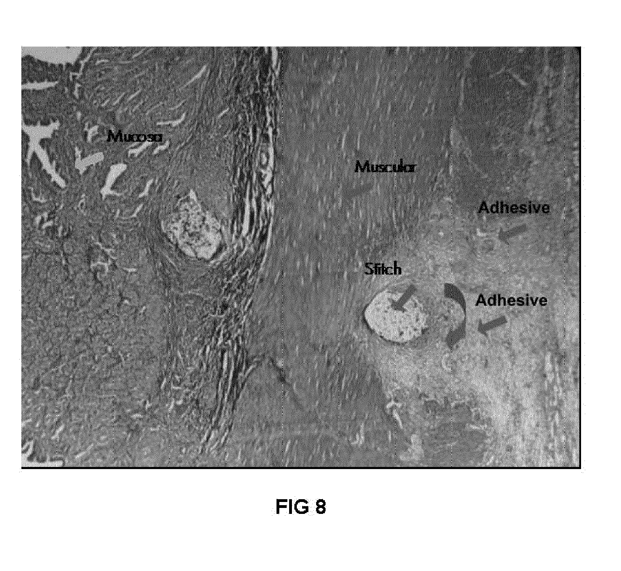

Careful histological evaluation has shown that in these experiments, the biological adhesive after being applied, penetrates rapidly through the serosal defects of the suture lines and advances in a few days to the entire intestinal wall reaching he mucosa where the process of metabolization occurs as shown in Figures 7,8 and 9. In Fig 7 (Masson stain) penetrates rapidly and is seen in the submucosa in the first 24 h after the application. There is no inflammatory reaction in the area of application of the fat pad to the serosa. In Figure 8, it can be seen that the biological adhesive penetrates and after 17 days disappears.

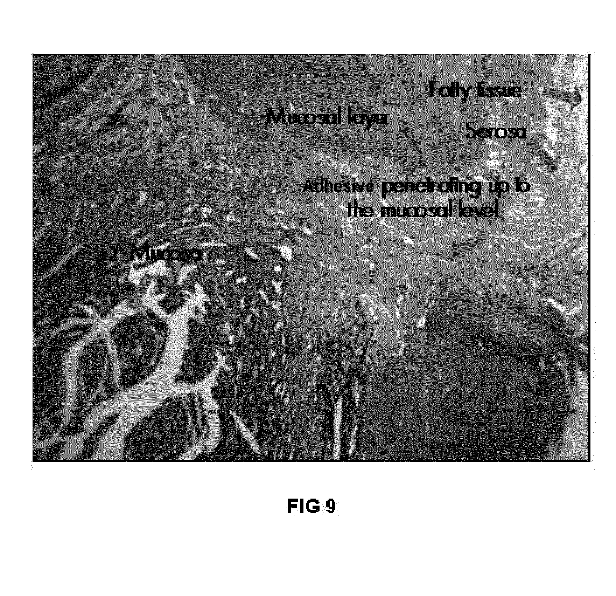

In Fig 9, (Mason stain), the biological adhesive is seen penetrating from the serosa to the intestinal wall full-thickness 11 days after the application. It probably disappears when the a-amylases metabolize the polymer and in 2-3 days disappears completely without any structural change. After 2 weeks the adhesive -after the critical days of healing have gone by-, is rarely seen though the fat patch is firmly attached to the suture serosal surface.

In other aspect of the invention, the biological adhesive placed freely in the serosal surface of intestinal loops, disappears in a few hours and does not produce any adhesion between the neighboring loops of intestine, which does have important clinical repercussion for it will not produce later on, the well - known complication of bowel obstruction when the amounts of the adhesive are small and a fat patch has been applied.

The use of such fat pad opatches has been common for decades as a way of ¨protecting¨ anastomosis and suture lines although a definite proof of its value is lacking. The fat patch as indicated before, becomes firmly attached to the suture lines and in a few days cannot be detached from the suture line and in no way interferes with the movility and function of the intestines. After a few days the patch cannot be detached from its place, and if a stronger force is applied, tears will be produced , even to the full-thickness of the intestine. This is not observed when the fat patch was applied without the biological adhesive.

In these experiments it was also found that fat pad patches were more useful and advantageous that cellulose or collagen patches.

In another embodiment of the present invention, some fistulas complications of a an anastomotic leakage treated conservatively -without surgery-, or recurrent as in the case of a complicated gastric bypass, can also be occluded from the internal orifice with an endoscopic procedure without the need of a reoperation.

The entire disclosure of all applications, patents and publications, cited above and below, are hereby incorporated by reference.

### EXAMPLE

The following examples are provided solely to illustrate the present formulation, and are not intended to limit the scope of the invention.

### Equipment utilized

- A Mettler Toledo model SW (Max 75 kg/150 lb; Min 0.01 Kg/0.02 lb)
- A mixing agitator Eurostar Power b IKA-WERKE (50-2000 1/min)
- A middle sized serrated propeller
- A stainless rustproof iron container
- A standard potentiometer
- A Brookfield viscosimeter

Procedure :
1. In the stainless iron container, in a solution of purified distilled water, 30 % of its weight the maltodextrin was added (55 % its weight) mixing it.
2. In the manufacturing process , a 14,9 % of its weight, zinc oxide was added increasing the velocity of the mixing process.

Appearance : resting state : A solid-like gel. Nce is prepared for its use: Gel
Color: Creamy white
pH : 6.9
Viscosity: resting state: 102,000 cp. Once preapred for clinical use: 19,500 cp .
Note: The process of reparing the biological adhesive for clinical use consists in applying external energy , mechanical (stirring) or thermic with a container with water at 40 o C both in less than a minute.

### RESEARCH SUPPORT OF THE INVENTION

### PROTOCOL APROVED BY THE SCHOOL OF MEDICINE, UNIVERSIDAD ANAHUAC, MEXICO CITY, FEBRUARY 2005:

### PHASE 1

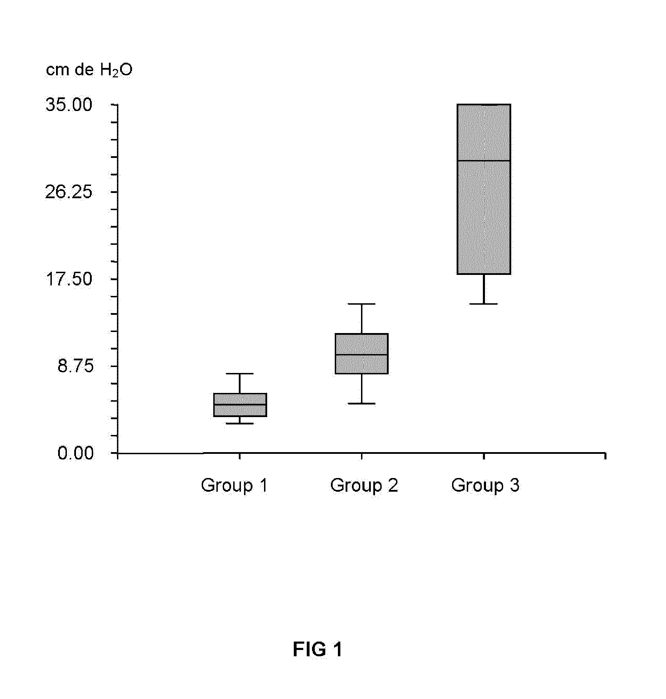

In the first phase, 250 to 300 g rats were used in acute experiments, in which, after an abdominal midline incision, 2 intestinal loops of 10 cm each were isolated with 4-0 silk sutures. Then, a 3 mm incision was made in the antimesenteric edge, sutured with a single stitch of 6-0 silk. At the ends of the closed loop, a 16 gauge intravenous catheter was introduced and connected "in a Y" fashion to a central venous pressure manometer and physiological serum was injected with a syringe, a method similar to the one described by Arnold et al. ⁶¹. Figure 1 graphically shows the hydraulic pressure required to produce leakage in the sutured line in each group (1, 2 and 3). In group 1 the hydraulic pressure necessary to produce leakage ("bursting pressure") in the sutured area, ranged from 3 - 5 cm of water, whereas in group 2 in which a patch of collagen of 3 mm² had been added, the hydraulic pressure necessary to produce such a leak increased to 5 - 8 cm of water. In group 3 the biological adhesive of the present invention was added to the patch, which was let to dry 15 minutes. The pressures that were required to produce the leakage of liquid in group 3 increased to 18 - 45 cm of water, which was a statistically significant difference p: 0.05. (test ANOVA). In each group 28 measurements were made.

In conclusion, in this phase 1, the biological adhesive of the present invention had a protective effect on the suture line, as demonstrated by a much higher hydraulic pressure being required to produce fluid leakage in group 3, as compared to groups 1 and 2.

### PHASE 2:

In sub acute experiments and using sterile techniques, in 250 to 300 g rats, a 2 to 3 cm midline vertical incision was made and an intestinal loop was exteriorized. An incision of 3 mm was made in the antimesenteric edge. In group 1, a patch of collagen of 3 mm² was placed over the incision but without any suture or adhesive. In group 2, a patch of collagen of 3 mm², to which an effective amount of the adhesive of the present invention was applied on the surface, was placed over the incision. The abdominal incision was sutured using 4-0 polidioxanone in two layers. The rats were returned to their cage and the survivors were observed for three weeks. The survival rate in group 1 was 52%, whereas in group 2 the survival rate was 75%.

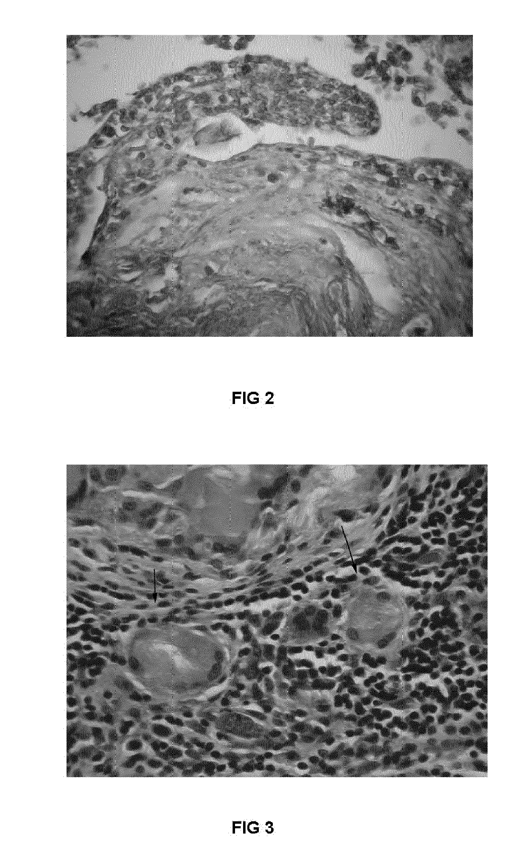

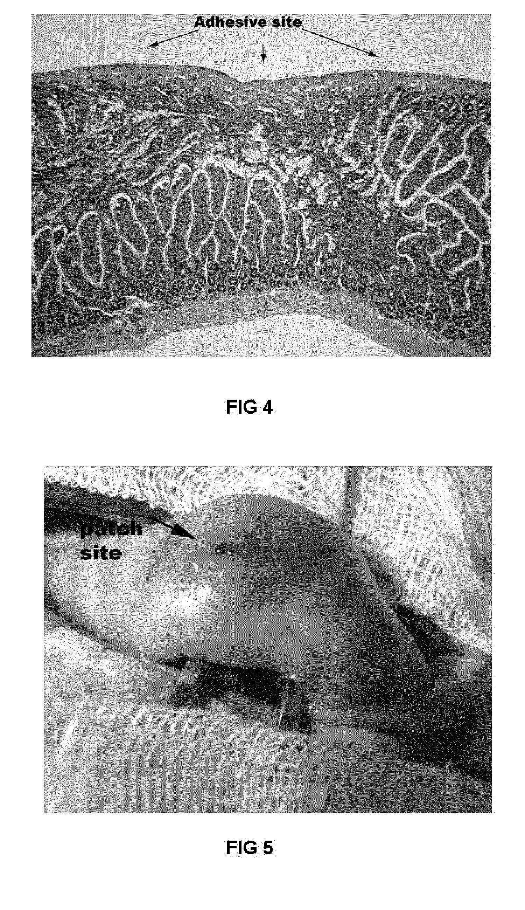

The purpose of phase 2 was to observe the tissue reaction, both macroscopically and microscopically, in the intestinal tissue. As can be seen in Figures 2 and 3, in the case of rats of group 1 that died in the first three days due to peritonitis, the well-known phenomena of intense inflammatory reaction, cellular infiltrate and even intestinal defect were observed (Figure 2). In the necropsies of the surviving rats in the same group, from the macroscopic point of view, multiple adhesions were observed to the abdominal wall and to the surrounding intestinal loops with fibrosis, and the presence of giant cells was also observed. In 2 cases large parietal abscesses were seen (Figure 3). On the other hand, in the surviving rats of group 2, it was impressive to observe an intact serous surface, and it was difficult or impossible to determine the site where there had been a laceration and patch with adhesive of the present invention. Microscopically, the intestinal wall healed normally (Figure 4), and in these animals no inflammatory reaction or giant cells were observed, which makes us conclude that the adhesive of the invention is very well tolerated and disappears after several days. As for the collagen patch used frequently in general surgery, orthopedic surgery, neurological surgery, etc., it is known that its reabsorption happens in the first 3 to 5 days after its application.

As a result of the experimental observations in phase 2, it is concluded that the biological adhesive of the present invention was very well accepted by intestinal tissues of the rat and sometimes it was impossible to determine with certainty the exact site of the application of the patch with adhesive in the site of the injury.

### PHASE 3:

The observations made of Phases 1 and 2, were extended and applied to dogs using sterile techniques. Preoperatively, 1g intramuscular of a second generation cephalosporin was applied. The technique involved making a 6 cm vertical midline incision under the xyphoid appendix, and, by means of careful gastric traction, the duodenum was exteriorized. Then, a 5 to 6 mm² laceration was made in the antimesenteric edge using a fine haemostatic clamp 4-5 cm distal to the pancreatic duct. Then, a 3 cm² patch of cellulose or collagen was applied with the adhesive of the present invention over the laceration. After 12 min., the duodenum was returned to its normal position, that is, the laceration was not sutured, and a patch with the adhesive was applied over the defect. The incision was then closed with 2-0 polidioxanone, a continuous suture in two separated layers.

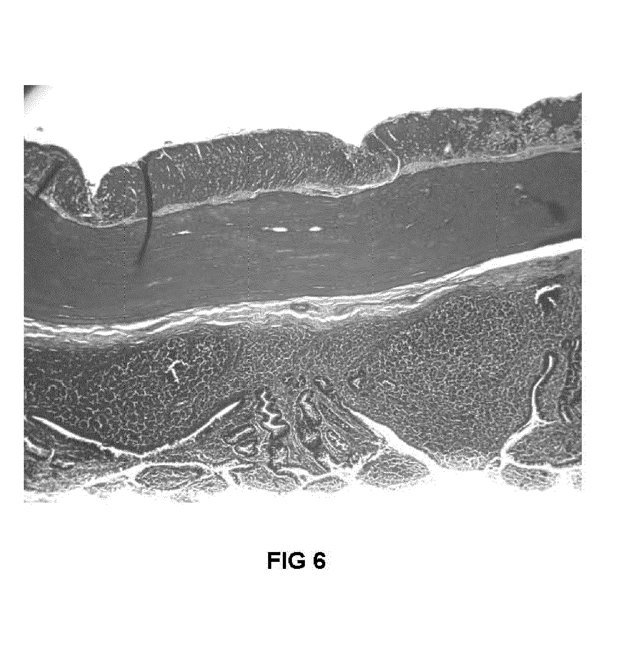

The animals were observed during 3 weeks and were sacrificed with an intravenous dose of sodic pentotal. In two occasions the autopsy took place 2 months later. Of a total of 28 dogs, 3 died in the first 72 hours, the first 2 and 1 with an extended laceration. The macroscopic evaluation, in four surviving animals demonstrated minimum adhesions to fatty tissue. But, in the rest of the animals, at the site or the injury, only a small scar was observed (Figure 5). The microscopic sections, shown in Figure 6, like in the previous phase 2 in rats, revealed a normal healing process of the intestinal wall, without inflammatory reaction or giant cells.

### PHASE 4:

Both in rats and in dogs the suture lines and the defects created in the duodenum and distal intestine not sutured but patched, were tested with hydraulic pressure at 8 days and the results were again the same: The biological adhesive of the present invention provided more strength to prevent "bursting" of the suture lines or defects as compared to the sites sutured and/or patched without adhesive formulation.

In conclusion, the biological adhesive of the present invention temporally protects the suture lines based on the fact that a greater hydraulic pressure in Phase 1 is required to produce leakage of liquid in the suture line when the patch and adhesive were placed. This results in a greater survival rate in rats as shown in Phase 2. In Phase 2, there was an intense inflammatory reaction and cellular infiltrate in rats which died soon (Phase 2, group 1: patch alone). A chronic inflammatory process with giant cells was observed in surviving rats (Phase 2, group 1: patch alone), as compared with a normal healing process when the patch was applied with the adhesive of the present invention (Phase 2, group 2: patch with adhesive). In Phase 3, involving dogs with a very serious, lethal injury in the duodenum, where it is known that pancreatic and intestinal secretions exist, most animals survived and similar findings to those observed in rats were present, namely a normal healing process in the duodenal wall and site of the injury. All of these results are proof that the adhesive of the present invention produces the necessary seal by its adhesive properties. In Phase 2 and in hase 3, the macroscopic and microscopic findings were similar: the biological adhesive is very well tolerated by the intestinal tissues, external adhesions practically do not exist, and histological signs of inflammation or giant cells were not observed. In Phase 2, it was practically impossible sometimes to determine the site of the injury in the intestine of the rat, and in the dogs in Phase 3, only a small scar existed. Microscopically, an inflammatory reaction or a foreign body type of reaction did not exist. All of these results lead to the conclusion that the adhesive of the present invention is not only very well tolerated, but is also biodegraded in 5 to 8 days after the application, precisely the critical period of intestinal healing in the case of the mentioned gastrointestinal anastomosis.

Finally, in both, rats and in dogs using a similar experimental method of Phases 1 and 3 at 8 days postoperatively, equal results were found: greater hydraulic pressures were required to produce leaks ("bursting pressure") in the suture lines and sites of defects patched when the adhesive of the present invention was applied.

### Experimental Comparative Study:

In order to compare the protection provided in suture lines by the biological adhesive , the following was carried out as a comparative example: In the same dog two 5 cm suture lines were produced using Vycryl 00 after wounds were made transversally. Both were carefully inspected and the adhesive was applied in the proximal one , and in both a fat patcha was applied and fixed distally with a single stitch of Vicryl 000. The loop was left outside the abdominal cavity during 15 minutes and then it was carefully returned to the peritoneal cavity and the incision was closed with a single continous suture using Vicryl 00 and the skin closed with a single continous layer of poliglecaprona 25. All animals were for by an experienced veterinarian and on the 7 th postoperative day and under general endotracheal anesthesia and using the same incision the abdomen was explored, the intestinal loop was again exteriorized and straight clamps were applied to oclude proximally and distally - and in the middle too-, and bursting pressure was measured with a standard manometer.

In the proximal suture line (with the adhesive of the present invention), it was found that burst pressures require were greater as compared with the distal suture lines (without the adhesive of the present invention), and additionally in proximal lines the saline leakage was seen mostly in the ends of the intestine clamped, whereas in the distal suture line (without the adhesive of the present invention) , leakage at a lesser pressure was seen in the area of the wound and suture line.

These findings correlate well with the observations made in other phase that in the 7 th postoperative day, the fat patches over the suture lines could not be detached from the suture lines where the adhesive of the present invention had benn applied lest a considerable tear was made even to the lumen of the intestine.

In cases where the biological adhesive leaked outside the operative site, no adhesions were found among the bowel loops, and in fact it was also found that the biological adhesive disappeared 2-3 hours after its application. On the other hand, and as mentioned before, when the biological adhesive was applied with a fat patch, a very firm seal was observed in the suture lines that conceivably added more protection to them.

Although a form of specific accomplishments of the present invention have been described in detail with the previous examples, it must be stated that the present invention and formulations, are susceptible of diverse modifications and alternative forms of use, without separating them from the spirit and reaches of the present invention. Therefore, the intention is not to limit the invention and method herein described, but instead, to cover all the equivalent and/or alternative modifications that fall within the reaches of the invention as it calls to each other by the annexed claims.

The preceding examples can be repeated with similar success by substituting the generically or specifically described reactants and/or operating conditions of this invention for those used in the preceding examples.

From the foregoing description, one skilled in the art can easily ascertain the essential characteristics of this invention and, without departing from the spirit and scope thereof, can make various changes and modifications of the invention to adapt it to various usages and conditions.

### REFERENCES

1. Schauer P. Gastric bypass for severe obesity: Approaches and outcomes. Surgery for Obesity and related Diseases. 2005, May - Jun; 1 (3): 297-300.
2. Buchwald H. Consensus Conference Statement: Bariatric surgery for morbid obesity: Health implications for patients, health professionals, and third-party payers. Surgery for Obesity and related Diseases, 2005 May-Jun; 1 (3): 371-381.
3. Jensen C, Flum DR. The costs of non-surgical and surgical weight loss interventions: Is an ounce of prevention really worth a pound of cure?. Surgery for Obesity and related Diseases. 2005 May-Jun; (1)3: 353-357.
4. Gastrointestinal surgery for severe obesity: National Institutes of Health Consensus Development Conference Statement. 1992 Feb; (2 Suppl): 615S-619S. Washington D.C.
5. Gagner M, et al., Chapter 26, Section III. Laparoscopic Roux-in-Y Bypass for morbid obesity, in: "Laparoscopic Surgery" 2003, Eds. Cueto J, Jacobs M, Gagner M, Ed. McGraw-Hill, New York.
6. Brolin R.E. Postoperative complications in the context of risk: benefit. Surgery for Obesity and related Diseases. 2005 May-Jun; 1(3): 343-347.
7. Gonzalez R, Nelson LG, et al. Anastomotic Leaks after Laparoscopic Gastric Bypass. Obesity Surgery. 2004 Nov-Dec; 14(10): 1299-1307.
8. Baker RS, Foote J, et al. The Science of Stapling and Leaks. Obesity Surgery. 2004 Nov-Dec; 14(10): 1290 - 1298.
9. Csendes A, Burdiles P, et al. Conservative Management of Anastomotic Leaks after 557 Open Gastric Bypasses. Obesity Surgery. 2005 Oct; 15(9): 1252 - 1256.
10. Truong S, Bohm G, et al. Results after endoscopic treatment of postoperative upper gastrointestinal fistulas and leaks using combined Vicryl plug and fibrin glue. Surgical Endoscopy. 2004 Jul;18(7): 1105-1108.
11. Sapala JA, Wood MH, et al. Anastomotic Leak Prophylaxis Using a Vapor-Heated Fibrin Sealant: Report on 738 Gastric Bypass Patients. Obesity Surgery. 2004 Jan; 14(1): 35-42.
12. Shikora SA. The Use of Staple-Line Reinforcement During Laparoscopic Gastric Bypass. Obesity Surgery. 2004 Nov-Dec; 14(10): 1313 - 1320.
13. Meng G y cols. Use of Fibrin Sealant in Laparoscopic Gastric Bypass for the Morbidly Obese, Obesity Surgery, 2004, 14-10, 1321-1327.
14. Plasencia J y cols, Laparoscopic Colectomy, Chapter 41, Section VI. Large Bowel Surgery, en Laparoscopic Surgery. 2003, Eds. Cueto J, Jacobs M, Gagner M. Ed. McGraw-Hill, New York.
15. Franklin M y cols. Colonic Resection in the Treatment of Colorectal Carcinoma: Multicentric Study with Prospective Comparison of the Traditional and Laparoscopic Methods, Chapter 42, Section VI, Large Bowel Surgery en "Laparoscopic Surgery" 2003. Eds. Cueto J, Jacobs M, Gagner M. Ed. McGraw-Hill, New York.
16. Appendix: Conclusions of the Consensus of the European Association for Endoscopic Surgery Experts Opinion Conference on Colonic Cancer by Elective Surgery, Chapter 43, Section VI, Large Bowel Surgery, en Laparoscopic Surgery. 2003, Eds. Cueto J, Jacobs M, Gagner M. Ed. McGraw-Hill, New York.
17. Birdas T y cols. Laparoscopic Colectomy for Benign Disease, Chapter 44 Section VI Large Bowel Surgery, en Laparoscopic Surgery.2003 Eds. Cueto J, Jacobs M, Gagner M. Ed. McGraw-Hill, New York.
18. Hartmann R y cols. Laparoscopic Surgery for Diverticular Disease, Chapter 45 Section VI, Large Bowel Surgery, en Laparoscopic Surgery. 2003, Eds. Cueto J, Jacobs M, Gagner M. Ed. McGraw-Hill, New York.
19. Gòmez E y cols. Laparoscopic Management of Colonic Emergencies, Chapter 47, Section VI, Large Bowel Surgery, en Laparoscopic Surgery. 2003 Eds. CuetoJ, Jacobs M, Gagner M. Ed. McGraw-Hill, New York.
20. Franklin M y cols . Complications of Laparoscopic Colorectal Surgery, Chapter 93, Section XVII, Complications of Laparoscopic Surgery en Laparoscopic Surgery. 2003. Eds. JCueto J, Jacobs M, Gagner M. Ed. McGraw-Hill, New York.
21. Nelson H, et al. A comparison of Laparoscopically assisted and open colectomy for colon cancer. The New England Journal of Medicine. 2004 May 13;350(20): 2050-2059.
22. Walker KG, Bell SW, et al. Anastomotic leakage is predictive of diminished survival after potentially curative resection for colorectal cancer. Annals of Surgery. 2004 Aug;240(2): 255-259.
23. Schardey HM, Joosten U, et al. The prevention of anastomotic leakage after total gastrectomy with local decontamination. A prospective, randomized, double-blind, placebo-controlled multicenter trial. Annals of Surgery. 1997 Feb; 225(2): 172-180.
24. Schardey HM, Krämling HJ, et al. Risk factors and pathogenic microorganisms in patients with insufficient esophagojejunostomy after gastrectomy. Zentralblatt Chirurgie. 1998;123(1):46-52.
25. Cueto J, Weber A. Laparoscopic reoperations in the esophageal hiatus: Cemalettin Topuzly, Yaman Tekant. "Proceedings: Joint Euro Asian, Congress of Endoscopic surgery" 5th Annual Congress of The European Association for Endoscopic Surgery, Monduzzi Editore,17 al 21 de Junio de 1997 . Estambul, Turquía. pag. 133.
26. Cueto J, Díaz O, et al. The efficacy of laparoscopic surgery in the diagnosis and treatment of peritonitis: Experience with 107 cases in Mexico City. Surgical Endoscopy. 1997 Apr; 11 (4):366-370.
27. Cueto J.Peritonitis Séptica. Medical Association of The American British Cowdray Hospital. Anales Médicos. September 1978; 23: 130-142.
28. Cueto J, Weber A, et al. Laparoscopic treatment of perforated duodenal ulcer. Surgical Laparoscopy & Endoscopy. 1993 Jun; 3(3): 216-218.
29. Cueto J et al. The Laparoscopic Treatment of a Perforated Peptic Ulcer, Chapter 21, Section III, Gastric Surgery, en: Laparoscopic Surgery. 2003, Eds. Cueto J, Jacobs M, Gagner M, Ed. McGraw-Hill, New York.
30. Kraus TW, Mehrabi A, et al. Scientific evidence for application of topical hemostats, tissue glues and sealants in hepatobiliary surgery. Journal of the American College of Surgeons. 2005 Mar; 200(3): 418-427.
31. Ninan L, Monahan J, et al. Adhesive strength of marine mussel extracts on porcine skin. Biomaterials. 2003 Oct; 24(22):4091-4099.
32. Lillemoe KD, Cameron JL, et al. Does fibrin glue sealant decrease the rate of pancreatic fistula after pancreaticoduodenectomy? Results of a prospective randomized trial. Journal of Gastrointestinal Surgery. 2004 Nov; 8(7): 766-772; discussion 772-4.
33. Cueto J, Sánchez J, et al. Cateterización percutánea y corrección de una fístula duodenal lateral. AND Revista Mexicana de Radiología, 1985; 39(2): 84-87.
34. Felix E. Laparoscopic Inguinal Hernioplasty, Chapter 62 Section IX, Hernia Surgery, en: Laparoscopic Surgery. 2003 Eds. Cueto J, Jacobs M, Gagner M, Ed. McGraw-Hill. New York.
35. Felix E, et al. Complications of Laparoscopic Inguinal Hernia Repair, Chapter 94 Section XVII, Complications of Laparoscopic Surgery, en Laparoscopic Surgery. 2003, Eds. Cueto J, Jacobs M, Gagner M, Ed. McGraw-Hill. New York.
36. Katkhouda N. A new technique for Laparoscopic hernia repair using fibrin sealant. Surgical Technology International. 2004; 12:120-126.
37. Kapischke M, Prinz K, et al. Precoating of alloplastic materials with living human fibroblasts: a feasibility study. Surgical Endoscopy. 2005 Jun; 19(6): 791-797.
38. D.J. Mandley, J.F. Birch, S.L. Williams, P.J. Trotter, F. Wilkinson and G.A. Davies. "Photon activated biological adhesives in surgery", International Journal of Adhesion and Adhesives 2000, 20(2), 97-102.
39. Birch JF, Mandley DJ, Williams SL,y cols. Methylene blue based protein solder for vascular anastomosis: an in vitro burst pressure study. Lasers in Surgery and Medicine 2000, 26, 323-329.
40. Halpern B. U.S. Patent No. 3,667,472 .titled "Adhesive for Living Tissue".. June 6, 1972,
41. WJohnston et al. Fibrin glue v sutured bolster: lessons learned during 100 Laparoscopic partial nephrectomies. J Urol. 2005 Jul;174(1):47-52.
42. Buchta C, Dettke M, et al. Impact of manufacturing, irradiation and filtration steps to bacterial contamination of autologous fibrin sealant. Biologicals. 2004 Sep; 32(3): 165-169.
43. Kawamura M, Gika M, et al. The sealing effect of fibrin glue against alveolar air leakage evaluated up to 48 h; comparison between different methods of application. European Journal of Cardio-thoracic Surgery. 2005 Jul;28(1):39-42.
44. UNI-BOND 1100D MATERIAL SAFETY DATA SHEET, Uniplast, Inc., UNI-BOND 1100D DATE PREPARED: July 8, 2000.
45. Furia T.E. CRC Handbook of food additives, Cleveland, Ohio. 1968.
46. Severian D. Polymeric Biomaterials Second Edition Revised Expanded); Chapter 1: Polysacharides as biomaterials, New York, NY, USA: Marcel Dekker Incorporated, 2001. pp 3- 30.
47. Spotnitz D, Burks S, and Mercer D y cols. Clinical Indications for Surgical Tissue Adhesives. Clinical Applications of tissue adhesives. Tissue Engineering and Biodegradable Equivalents: Scientific and Clinical Applications, (Eds.) Journal of Controlled Release Volume 92, Issue 3, October 30, 2003, Pages 399-400, Marcel Dekker Incorporated, New York, 2002, pp. 652-660.
48. Thornton M, Barkley L, et al. Anti-Kaposi's Sarcoma and Antiangiogenic Activities of Sulfated Dextrins. Antimicrob Agents Chemothe. 1999 Oct;43(10):2528-2533.
49. Wiggins KJ, Rumpsfeld M, et al. Predictors of a favourable response to icodextrin in peritoneal dialysis patients with ultrafiltration failure. Nephrology (Carlton, Vic). 2005 Feb; 10(1): 33-36.
50. Peers EM, Brown and Adept Adhesion Study Group Effectiveness of 4% Icodextrin in a Pivotal Adhesion Reduction Trial in the USA o ABSTRACT. Fertility and Sterility, Volume 84, Supplement 1, September 2005, Page S155.
51. Goldsmith D, Jayawardene S, et al. Allergic reactions to the polymeric glucose-based peritoneal dialysis fluid icodextrin in patients with renal failure. Lancet. 2000 Mar 11;355(9207): 897.
52. Van den Tol P, Ten Raa S, et al. Icodextrin reduces postoperative adhesion formation in rats without affecting peritoneal metastasis. Surgery. 2005 Mar; 137(3): 348-354.
53. Almeida Prieto S, Blanco Méndez J, et al. Starch-dextrin mixtures as base excipients for extrusion-spheronization pellets. European Journal of Pharmaceutics and Biopharmaceutics. 2005 Apr; 59(3): 511-521.
54. el-Mougi M, Hendawi A, et al. Efficacy of standard glucose-based and reduced-osmolarity maltodextrin-based oral rehydration solutions: effect of sugar malabsorption. Bulletin of the World Health Organization. 1996;74(5):471-477.
55. Jungheinrich C, Scharpf R, et al. The Pharmacokinetics and Tolerability of an Intravenous Infusion of the New Hydroxyethyl Starch 130/0.4 (6%, 500 mL) in Mild-to-Severe Renal Impairment. Anesthesia and Analgesia. 2002 Sep; 95(3):544-551.
56. Nelson JL, Kuzman JH, et al. Intraperitoneal lavage and kanamycin for the contaminated abdomen. The Surgical Clinics of North America. 1975 Dec;55(6): 1391-1395.
57. Yelon JA, Green JD, et al. Efficacy of an intraperitoneal antibiotic to reduce the incidence of infection in the trauma patient: a prospective, randomized study. Journal of the American College Surgeouns. 1996 Jun;182(6):509-514.
58. Gorman T, Eisele G, et al. Intraperitoneal antibiotics effectively treat non-dialysis-related infections. Peritoneal Dialysis International. 1995 Jul-Sep;15(6):283-284.
59. Alexander JW. Wound Infections in the Morbidly Obese. Obesity Surgery. 2005 Oct; 15(9): 1276- 1277.
60. Alexander JW and Rahn R. Prevention of Deep Wound Infection in Morbidly Obese Patients by Infusion of an Antibiotic into the Subcutaneous Space at the Time of Wound Closure. Obesity Surgery. 2004 Aug; 14(7): 970-974.
61. Arnold W and Shikora SA. A Comparison of Burst Pressure Between Buttressed Versus Non-Buttressed Staple-Lines in an animal Model. Obesity Surgery. 2005 Feb; 15(2): 164-171.
62. Fielding LP, Stewart-Brown S, et al. Anastomotic integrity after operations for large-bowel cancer: a multicentre study. British Medical Journal. 1980 Aug 9;281 (6237):411-414.
63. Fielding LP, Stewart-Brown S, et al. Covering Stoma for Elective Anterior Resection of the Rectum: An Outmoded Operation?. The American Journal of Surgery. 1984 Apr;147(4):524-530.
64. Zaporozhets AA. Physical and biologic impermeability of intestinal sutures in the first twenty-four hours after operations on the gastrointestinal tract. Surgery. 1992 Nov;112(5):940-945.
65. Wasserberg N, Tzakis AG, et al. Anastomotic healing in small bowel transplantation model in the rat. World Journal of Surgery. 2004 Jan;28(1):69-73.
66. Keighley MR. Prevention of infection in surgical wounds. Annals of the Royal College of Surgeons of England. 1978 May;60(3):243-245.
67. te Velde EA, Voest EE, van Gorp JM, Verhemm A, et al., Adverse effects of the antiangiogenic agent angiostatin on the healing of experimental colonic anastomosis. Ann Surg Oncol 2002;9:303-9;
68. Rizk M, Witte MB, Et al. Nitric oxide and wound healing. World Journal of Surgery 2004 Mar;28(3):301-306.
69.Phinney RB, Schwartz SD, Lee, et al. Collagen-shield delivery of gentamycin and vancomycin. Archives of Ophthalmology, 1988 Nov;106(11):1599-1604.
70.Bennet NT, Schultz GS, Growth factors and wound healing: Part II. Role in normal and chronic wound healing. Am J Surg 1993;166:74-81
   -O'Leary DP. Use of the greater omentum in colorectal surgery. Diseases of the Colon and Rectum. 1999 Apr;42(4):533-539.
   Saber AA and Jackson O. Omental wrap: A simple technique for Reinforcement of the gastrojejunostomy during Roux-en-Y gastric bypass. Obesity Surgery. 2007 Jan;17(1):15-18.
   -Cueto J, Weber A, et al. Laparoscopic treatment of perforated duodenal ulcer. Surgical Laparoscopy and Endoscopy. 1993 Jun;3(3):216-218.
   -Finsterbush A, Frankl U, et al. Fat pad adhesion to partially torn anterior cruciate ligament: a cause of knee locking. The American Journal of Sports Medicine. 1989 Jan-Feb;17(1):92-95.
   -Kawashima O, Hirai T, Kamiyoshihara M y cols. Use of a Pericardial Adipose tissue patch for Alveolar Air Leaks After Pulmonary Resections. Asian Cardiovasc Thorac Ann 1998;6:115-117.
   -Black P. Cerebrospinal fluid leaks following spinal surgery: use of fat grafts for prevention and repair. Technical Note. Journal of Neurosurgery. 2002 Mar;96(2 Suppl):250-252.
   -Figueras J, Llado L, et al. Application of fibrin glue sealant after hepatectomy does not seem justified: results of a randomized study in 300 patients. Annals of Surgery. 2007 Apr;245(4):536-542.
   -Itani KM, Wilson SE, et al. Ertapenem versus cefotetan prophylaxis in elective colorectal surgery. The New England Journal of Medicine. 2006 Dec 21;355(25):2640-2651.
   Ahrendt GM, ]Gardner K, et al. Loss of colonic structural collagen impairs healing during intra-abdominal sepsis. Archives of Surgery. 1994 Nov;129(11):1179-1183.

## Claims

**1.** A biological thixotropic adhesive to seal and protect anastomosis and suture lines temporarily in the internal body cavities , the adhesive containing dextrin as an element of dispersion , where the biological adhesive contains a a structural component that provides thixotropy to such adhesive , where such formula contains 80-97 % of its weight as dextrin in basis of the total weight , as element of dispersion and the dextrin itself contains from 45-75 % of solid contents and where the biological thixotropic adhesive has a viscosity above 100,000 cp in resting state and an application viscosity in the range of 15,000 - 27,000 cp, a pH of 6.5-7.5 a TGA 61.12 and has an adhesivity of 6 Mpa

**2.** A biological thixotropic adhesive according to claim 1 in which the dispersion is dispersion is made of a dextrin mixed with water.

**3.** A biological thixotropic adhesive according to claim 1, in which the adhesive contains from 88-92 % of its weight by the dispersion agent, based on the total weight of the composition.

**4.** A biological thixotropic adhesive according to claim 1, in which the dextrin dispersion has a solid content of 60-68 %.

**5.** A biological thixotropic adhesive according to claim 1, in which the dextrin has a dextrose equivalent (DE) of 10-11.

**6.** A biological thixotropic adhesive according to claim 1, in which the dextrin has a pH of 4-7 in a solution at 20 %.

**7.** A biological thixotropic adhesive according to claim 1, in which the dextrin used in the adhesive is a white powder, soluble, slightly sweet, non-hygroscopic with a density of 0.5 - 0.6 g/ml.

**8.** A biological thixotropic adhesive according to claim 1, in which the dextrin is a maltodextrin.

**9.** A biological thixotropic adhesive according to claim 1, in which the structural component provides thixotropy to the adhesive is an insoluble metal oxide.

**10.** A biological thixotropic adhesive according to claim 1, in which the structural component that provides thixotropy is a compound of calcium, iron, titanium, zirconium, cobalt or zinc.

**11.** A biological thixotropic adhesive according to claim 1, in which the structural component that provides thixotropy is zinc oxide.

**12.** A biological thixotropic adhesive according to claim 1, in which the structural component that provides thixotropy is present in a quantity between 10-12 % in the total weight of the adhesive.

**13.** A biological thixotropic adhesive according to claim 1, in which includes at least one antibiotic, being the antibiotic kanamycin.

**14.** A biological thixotropic adhesive according to claim 13, where the antibiotic is present in a quantity that caries between 0.1-1 % of the total weight of the adhesive.

**15.** A biological thixotropic adhesive according to claim 14, in which the antibiotic is present in a quantity that varies between 0.2-0.5 % of total weight of the adhesive.

**16.** A biological thixotropic adhesive according to claim 1, in which the adhesive contains up to 19 % of the structural component that provides thixotropy, and from o.1-1 % of total weight of an antibiotic, all based in the adhesive total weight.

**17.** A biological thixotropic adhesive according to claim 16, in which the adhesive contains from 10-20 % of its weight of a structural component that provides thixotropy to the adhesive, based in total weight of the adhesive.

**18.** A biological thixotropic adhesive according to claim 17, in which the structural component that provides thixotropic to the adhesive is zinc oxide and the antibiotic is kanamycin.

**22.** A biological thixotropic adhesive according to claim 1, that includes also an interphase of fatty tissue to avoid adhesions with the neighboring organs and structures.

**23.** A method to stimulate the process of healing in a patient and avoid adhesions with neighboring organs and structures that includes: applying to the tissue a biological thixotropic adhesive that contains dextrin as an element of dispersion, where the adhesive contains a structural component that provides thixotropy to the adhesive , where such formula contains 80-97 % of total weight of dextrin as an element of dispersion and the dextrin itself contains 45-75 % of solid content, and where the adhesive has a viscosity in resting state above 100,000 cp, and a viscosity at time of application in the range of 15,000-27,000 cp , a pH of 6.5-7.5 , a TGA of 61.12 and has an adhesivity at 24 h of 6 Mpa; and apply a patch with an interphase of fatty tissue over the biological thixotropic adhesive.
